# EUROPEAN PATENT APPLICATION

(11) **EP 4 056 697 A1**
(43) Date of publication of application: **14.09.2022**
(21) Application number: 21739525.0
(22) Date of filing: 23.04.2021
(51) Int. Cl.: C12N 9/78, C12N 15/77, C12P 13/14

(54) **NOVEL GLUCOSAMINE-6-PHOSPHATE DEAMINASE VARIANT, AND METHOD FOR PRODUCING L-GLUTAMIC ACID USING SAME**

(30) Priority: 27.01.2021 KR 20210011242
(71) Applicant: CJ Cheiljedang Corporation, Seoul 04560 (KR)
(72) Inventor: PARK, Goun, Seoul 04560 (KR); BAE, Jee Yeon, Seoul 04560 (KR); KWON, Nara, Seoul 04560 (KR); KIM, Hyo Jin, Seoul 04560 (KR); LEE, Jin Nam, Seoul 04560 (KR); SEO, Chang Il, Seoul 04560 (KR)
(74) Representative: Mewburn Ellis LLP
(86) International application number: PCT/KR2021/005154
(87) International publication number: WO 2022/163935

(57) **Abstract**

The present disclosure relates to a novel glucosamine-6-phosphate deaminase variant, a *Corynebacterium glutamicum* strain comprising the variant, and a method for producing L-glutamic acid using the strain.

<110>    CJ CheilJedang Corporation

<120>    Novel glucosamine-6-phosphate deaminase variant and a method for
         producing L-glutamic acid using the same

<130>    OPA21162-PCT

<150>    KR 10-2021-0011242
<151>    2021-01-27

<160>    19

<170>    KoPatentIn 3.0

<210>    1
<211>    253
<212>    PRT
<213>    Unknown

<220>
<223>    glucosamine-6-phosphate deaminase variant amino acid sequence


<400>    1
Met Glu Ile Thr Ile Cys Lys Asp Glu Gln Glu Val Gly Lys Ala Ala
  1               5                  10                  15

Ala Ala Leu Ile Ala Pro Phe Ala Asn Lys Gly Gly Thr Leu Gly Leu
             20                  25                  30

Ala Thr Gly Ser Ser Pro Leu Ser Thr Tyr Gln Glu Leu Ile Arg Met
         35                  40                  45

Tyr Glu Ala Gly Glu Val Ser Phe Lys Asn Cys Lys Ala Phe Leu Leu
     50                  55                  60

Asp Glu Tyr Val Gly Leu Thr Arg Asp Asp Glu Asn Ser Tyr Phe Lys
 65                  70                  75                  80

Thr Ile Arg Lys Glu Phe Thr Asp His Ile Asp Ile Val Asp Glu Glu
                 85                  90                  95

Val Tyr Ser Pro Asp Gly Ala Asn Pro Asp Pro Tyr Glu Ala Ala Ala
            100                 105                 110

Glu Tyr Glu Ala Lys Ile Ala Ala Glu Ser Val Glu Val Gln Ile Leu
        115                 120                 125

Gly Ile Gly Gly Asn Gly His Ile Val Phe Asn Glu Pro Ser Ser Ser
    130                 135                 140

Leu Ser Gly Leu Thr Lys Val Gln Ala Leu His Pro Lys Thr Val Glu
145                 150                 155                 160

Asp Asn Ala Arg Phe Phe Asn Thr Ile Glu Glu Val Pro Thr His Ala
                165                 170                 175

Val Thr Gln Gly Leu Gly Thr Leu Ser Arg Ala Gln Asn Ile Val Leu
            180                 185                 190

Val Ala Thr Gly Glu Gly Lys Ala Asp Ala Ile Arg Gly Thr Val Glu
        195                 200                 205

Gly Pro Val Thr Ala Ser Cys Pro Gly Ser Ile Leu Gln Met His Asn
    210                 215                 220

Asn Ala Thr Ile Ile Val Asp Glu Ala Ala Ala Ser Lys Leu Glu Asn
225                 230                 235                 240

Ala Asp His Tyr Arg Leu Met Glu Gln Leu Lys Leu Arg
                245                 250

<210>    2
<211>    762
<212>    DNA
<213>    Unknown

<220>
<223>    glucosamine-6-phosphate deaminase variant nucleotide sequence

<400>    2
atggaaatca ctatctgcaa agacgagcaa gaagtcggca aagcagctgc agccctaatc      60

gcacccttcg ccaacaaggg tggaaccttg gggcttgcaa caggatcctc accactgagt     120

acctaccaag agctcattcg catgtatgaa gctggggaag tgtcattcaa gaactgcaag     180

gcattcttgt tggatgaata cgtgggacta acccgtgacg atgaaaacag ctactttaaa     240

accattcgca aagagttcac tgaccacatc gacatcgttg atgaagaggt ctacagccca     300

gatggtgcaa accctgatcc atacgaagca gctgcagagt atgaggcaaa gatcgctgca     360

gaatccgttg aagttcaaat ccttggcatc ggcggaaacg gccacatcgt tttcaatgag     420

ccatcatctt ctctgtcagg actgacaaag gtccaggcgc tgcaccctaa aactgtggag     480

gacaacgctc gattcttcaa caccatcgaa gaggtcccaa cccacgccgt cacccagggt     540

ttgggcactt tgtcccgcgc gcaaaacatc gtgttggtgg caactggtga aggaaaagcc     600

gacgccatcc gcggaactgt ggaaggccca gtgactgctt cttgcccagg ttccatcctg     660

cagatgcaca acaatgccac catcatcgtt gatgaagcag cagcatccaa gctggaaaac     720

gctgatcact accgtctcat ggagcaatta aagctgcgct ag                        762

<210>    3
<211>    253
<212>    PRT
<213>    Unknown

<220>
<223>    glucosamine-6-phosphate deaminase amino acid sequence

<400>    3
Met Glu Ile Thr Ile Cys Lys Asp Glu Gln Glu Val Gly Lys Ala Ala
  1               5                  10                  15

Ala Ala Leu Ile Ala Pro Phe Ala Asn Lys Gly Gly Thr Leu Gly Leu
             20                  25                  30

Ala Thr Gly Ser Ser Pro Leu Ser Thr Tyr Gln Glu Leu Ile Arg Met
        35                  40                  45

Tyr Glu Ala Gly Glu Val Ser Phe Lys Asn Cys Lys Ala Phe Leu Leu
    50                  55                  60

Asp Glu Tyr Val Gly Leu Thr Arg Asp Asp Glu Asn Ser Tyr Phe Lys
65                  70                  75                  80

Thr Ile Arg Lys Glu Phe Thr Asp His Ile Asp Ile Val Asp Glu Glu
                85                  90                  95

Val Tyr Ser Pro Asp Gly Ala Asn Pro Asp Pro Tyr Glu Ala Ala Ala
            100                 105                 110

Glu Tyr Glu Ala Lys Ile Ala Ala Glu Ser Val Glu Val Gln Ile Leu
        115                 120                 125

Gly Ile Gly Gly Asn Gly His Ile Ala Phe Asn Glu Pro Ser Ser Ser
    130                 135                 140

Leu Ser Gly Leu Thr Lys Val Gln Ala Leu His Pro Lys Thr Val Glu
145                 150                 155                 160

Asp Asn Ala Arg Phe Phe Asn Thr Ile Glu Glu Val Pro Thr His Ala
                165                 170                 175

Val Thr Gln Gly Leu Gly Thr Leu Ser Arg Ala Gln Asn Ile Val Leu
            180                 185                 190

Val Ala Thr Gly Glu Gly Lys Ala Asp Ala Ile Arg Gly Thr Val Glu
        195                 200                 205

Gly Pro Val Thr Ala Ser Cys Pro Gly Ser Ile Leu Gln Met His Asn
    210                 215                 220

Asn Ala Thr Ile Ile Val Asp Glu Ala Ala Ala Ser Lys Leu Glu Asn
225                 230                 235                 240

Ala Asp His Tyr Arg Leu Met Glu Gln Leu Lys Leu Arg
                245                 250


<210>    4
<211>    762
<212>    DNA
<213>    Unknown

<220>
<223>    glucosamine-6-phosphate deaminase nucleotide sequence


<400>    4
atggaaatca ctatctgcaa agacgagcaa gaagtcggca aagcagctgc agccctaatc      60

gcacccttcg ccaacaaggg tggaaccttg gggcttgcaa caggatcctc accactgagt      120

acctaccaag agctcattcg catgtatgaa gctggggaag tgtcattcaa gaactgcaag      180

gcattcttgt tggatgaata cgtgggacta acccgtgacg atgaaaacag ctactttaaa      240

accattcgca aagagttcac tgaccacatc gacatcgttg atgaagaggt ctacagccca      300

gatggtgcaa accctgatcc atacgaagca gctgcagagt atgaggcaaa gatcgctgca 360

gaatccgttg aagttcaaat ccttggcatc ggcggaaacg gccacatcgc tttcaatgag 420

ccatcatctt ctctgtcagg actgacaaag gtccaggcgc tgcaccctaa aactgtggag 480

gacaacgctc gattcttcaa caccatcgaa gaggtcccaa cccacgccgt cacccagggt 540

ttgggcactt tgtcccgcgc gcaaaacatc gtgttggtgg caactggtga aggaaaagcc 600

gacgccatcc gcggaactgt ggaaggccca gtgactgctt cttgcccagg ttccatcctg 660

cagatgcaca acaatgccac catcatcgtt gatgaagcag cagcatccaa gctggaaaac 720

gctgatcact accgtctcat ggagcaatta aagctgcgct ag 762

<210> 5
<211> 35
<212> DNA
<213> Artificial Sequence

<220>
<223> primer 1F

<400> 5
tcgagctcgg taccctgact caaacggcca ggtgc 35

<210> 6
<211> 20
<212> DNA
<213> Artificial Sequence

<220>
<223> primer 2R

<400> 6
tcattgaaaa cgatgtggcc 20

<210> 7
<211> 20
<212> DNA
<213> Artificial Sequence

<220>
<223> primer 3F

<400> 7
ggccacatcg ttttcaatga 20

<210> 8
<211> 35
<212> DNA
<213> Artificial Sequence

<220>
<223> primer 4R

<400>    8
ctctagagga tcccctgctg gtagtagacg tggag                                35

<210>    9
<211>    20
<212>    DNA
<213>    Artificial Sequence

<220>
<223>    primer 5F

<400>    9
tgactcaaac ggccaggtgc                                                 20

<210>    10
<211>    20
<212>    DNA
<213>    Artificial Sequence

<220>
<223>    primer 6R

<400>    10
tgctggtagt agacgtggag                                                 20

<210>    11
<211>    38
<212>    DNA
<213>    Artificial Sequence

<220>
<223>    primer odhA_up_F

<400>    11
tgaattcgag ctcggtaccc ttgaacggaa ttgggtgg                             38

<210>    12
<211>    38
<212>    DNA
<213>    Artificial Sequence

<220>
<223>    primer odhA_up_R

<400>    12
cccaggtggc atcggtacct tcacccagcg ccacgcag                             38

<210>    13
<211>    39
<212>    DNA
<213>    Artificial Sequence

<220>
<223>    primer odhA_down_F


<400>    13
cgctgggtga aggtaccgat gccacctggg ttggtcaag                              39


<210>    14
<211>    38
<212>    DNA
<213>    Artificial Sequence

<220>
<223>    primer odhA_down_R


<400>    14
gtcgactcta gaggatcccc ggacaaggaa tggagaga                               38


<210>    15
<211>    18
<212>    DNA
<213>    Artificial Sequence

<220>
<223>    primer odhA_del_F


<400>    15
cttaccgttg ttgccctt                                                     18


<210>    16
<211>    19
<212>    DNA
<213>    Artificial Sequence

<220>
<223>    primer odhA_del_R


<400>    16
ctccttcacc cacatcatt                                                    19


<210>    17
<211>    1205
<212>    PRT
<213>    Unknown

<220>
<223>    OdhA amino acid sequence


<400>    17
Met Phe Gln Gln Phe Gln Lys Asp Pro Lys Ser Val Asp Lys Glu Trp
1               5                   10                  15

Arg Glu Leu Phe Glu Ala Gln Gly Gly Pro Asn Ala Thr Pro Ala Thr
            20                  25                  30

Thr Glu Ala Gln Pro Ser Ala Pro Lys Glu Ser Ala Lys Pro Ala Pro
        35                  40                  45

Lys Ala Ala Pro Ala Ala Lys Ala Ala Pro Arg Val Glu Thr Lys Pro
    50                  55                  60

Ala Ala Lys Thr Ala Pro Lys Ala Lys Glu Ser Ser Val Pro Gln Gln
65                  70                  75                  80

Pro Lys Leu Pro Glu Pro Gly Gln Thr Pro Ile Arg Gly Ile Phe Lys
                85                  90                  95

Ser Ile Ala Lys Asn Met Asp Ile Ser Leu Glu Ile Pro Thr Ala Thr
            100                 105                 110

Ser Val Arg Asp Met Pro Ala Arg Leu Met Phe Glu Asn Arg Ala Met
        115                 120                 125

Val Asn Asp Gln Leu Lys Arg Thr Arg Gly Gly Lys Ile Ser Phe Thr
    130                 135                 140

His Ile Ile Gly Tyr Ala Met Val Lys Ala Val Met Ala His Pro Asp
145                 150                 155                 160

Met Asn Asn Ser Tyr Asp Val Ile Asp Gly Lys Pro Thr Leu Ile Val
                165                 170                 175

Pro Glu His Ile Asn Leu Gly Leu Ala Ile Asp Leu Pro Gln Lys Asp
            180                 185                 190

Gly Ser Arg Ala Leu Val Val Ala Ala Ile Lys Glu Thr Glu Lys Met
        195                 200                 205

Asn Phe Ser Glu Phe Leu Ala Ala Tyr Glu Asp Ile Val Thr Arg Ser
    210                 215                 220

Arg Lys Gly Lys Leu Thr Met Asp Asp Tyr Gln Gly Val Thr Val Ser
225                 230                 235                 240

Leu Thr Asn Pro Gly Gly Ile Gly Thr Arg His Ser Val Pro Arg Leu
                245                 250                 255

Thr Lys Gly Gln Gly Thr Ile Ile Gly Val Gly Ser Met Asp Tyr Pro
            260                 265                 270

Ala Glu Phe Gln Gly Ala Ser Glu Asp Arg Leu Ala Glu Leu Gly Val
        275                 280                 285

Gly Lys Leu Val Thr Ile Thr Ser Thr Tyr Asp His Arg Val Ile Gln
    290                 295                 300

Gly Ala Val Ser Gly Glu Phe Leu Arg Thr Met Ser Arg Leu Leu Thr
305                 310                 315                 320

Asp Asp Ser Phe Trp Asp Glu Ile Phe Asp Ala Met Asn Val Pro Tyr
                325                 330                 335

Thr Pro Met Arg Trp Ala Gln Asp Val Pro Asn Thr Gly Val Asp Lys
            340                 345                 350

Asn Thr Arg Val Met Gln Leu Ile Glu Ala Tyr Arg Ser Arg Gly His
        355                 360                 365

Leu Ile Ala Asp Thr Asn Pro Leu Ser Trp Val Gln Pro Gly Met Pro
    370                 375                 380

Val Pro Asp His Arg Asp Leu Asp Ile Glu Thr His Ser Leu Thr Ile
385                 390                 395                 400

Trp Asp Leu Asp Arg Thr Phe Ser Val Gly Gly Phe Gly Gly Lys Glu
                405                 410                 415

Thr Met Thr Leu Arg Glu Val Leu Ser Arg Leu Arg Ala Ala Tyr Thr
            420                 425                 430

Leu Lys Val Gly Ser Glu Tyr Thr His Ile Leu Asp Arg Asp Glu Arg
        435                 440                 445

Thr Trp Leu Gln Asp Arg Leu Glu Ala Gly Met Pro Lys Pro Thr Gln
    450                 455                 460

Ala Glu Gln Lys Tyr Ile Leu Gln Lys Leu Asn Ala Ala Glu Ala Phe
465                 470                 475                 480

Glu Asn Phe Leu Gln Thr Lys Tyr Val Gly Gln Lys Arg Phe Ser Leu
                485                 490                 495

Glu Gly Ala Glu Ala Leu Ile Pro Leu Met Asp Ser Ala Ile Asp Thr
            500                 505                 510

Ala Ala Gly Gln Gly Leu Asp Glu Val Val Ile Gly Met Pro His Arg
        515                 520                 525

Gly Arg Leu Asn Val Leu Phe Asn Ile Val Gly Lys Pro Leu Ala Ser
    530                 535                 540

Ile Phe Asn Glu Phe Glu Gly Gln Met Glu Gln Gly Gln Ile Gly Gly
545                 550                 555                 560

Ser Gly Asp Val Lys Tyr His Leu Gly Ser Glu Gly Gln His Leu Gln
                565                 570                 575

Met Phe Gly Asp Gly Glu Ile Lys Val Ser Leu Thr Ala Asn Pro Ser
            580                 585                 590

His Leu Glu Ala Val Asn Pro Val Met Glu Gly Ile Val Arg Ala Lys
        595                 600                 605

Gln Asp Tyr Leu Asp Lys Gly Val Asp Gly Lys Thr Val Val Pro Leu
    610                 615                 620

Leu Leu His Gly Asp Ala Ala Phe Ala Gly Leu Gly Ile Val Pro Glu
625                 630                 635                 640

Thr Ile Asn Leu Ala Lys Leu Arg Gly Tyr Asp Val Gly Gly Thr Ile
                645                 650                 655

His Ile Val Val Asn Asn Gln Ile Gly Phe Thr Thr Thr Pro Asp Ser
            660                 665                 670

Ser Arg Ser Met His Tyr Ala Thr Asp Tyr Ala Lys Ala Phe Gly Cys
        675                 680                 685

Pro Val Phe His Val Asn Gly Asp Asp Pro Glu Ala Val Val Trp Val
    690                 695                 700

Gly Gln Leu Ala Thr Glu Tyr Arg Arg Arg Phe Gly Lys Asp Val Phe
705                 710                 715                 720

Ile Asp Leu Val Cys Tyr Arg Leu Arg Gly His Asn Glu Ala Asp Asp
                725                 730                 735

Pro Ser Met Thr Gln Pro Lys Met Tyr Glu Leu Ile Thr Gly Arg Glu
            740                 745                 750

Thr Val Arg Ala Gln Tyr Thr Glu Asp Leu Leu Gly Arg Gly Asp Leu
        755                 760                 765

Ser Asn Glu Asp Ala Glu Ala Val Val Arg Asp Phe His Asp Gln Met
    770                 775                 780

Glu Ser Val Phe Asn Glu Val Lys Glu Gly Gly Lys Lys Gln Ala Glu
785                 790                 795                 800

Ala Gln Thr Gly Ile Thr Gly Ser Gln Lys Leu Pro His Gly Leu Glu
                805                 810                 815

Thr Asn Ile Ser Arg Glu Glu Leu Leu Glu Leu Gly Gln Ala Phe Ala
            820                 825                 830

Asn Thr Pro Glu Gly Phe Asn Tyr His Pro Arg Val Ala Pro Val Ala
        835                 840                 845

Lys Lys Arg Val Ser Ser Val Thr Glu Gly Gly Ile Asp Trp Ala Trp
    850                 855                 860

Gly Glu Leu Leu Ala Phe Gly Ser Leu Ala Asn Ser Gly Arg Leu Val
865                 870                 875                 880

Arg Leu Ala Gly Glu Asp Ser Arg Arg Gly Thr Phe Thr Gln Arg His
                885                 890                 895

Ala Val Ala Ile Asp Pro Ala Thr Ala Glu Glu Phe Asn Pro Leu His
            900                 905                 910

Glu Leu Ala Gln Ser Lys Gly Asn Asn Gly Lys Phe Leu Val Tyr Asn
        915                 920                 925

Ser Ala Leu Thr Glu Tyr Ala Gly Met Gly Phe Glu Tyr Gly Tyr Ser
    930                 935                 940

Val Gly Asn Glu Asp Ser Val Val Ala Trp Glu Ala Gln Phe Gly Asp
945                 950                 955                 960

Phe Ala Asn Gly Ala Gln Thr Ile Ile Asp Glu Tyr Val Ser Ser Gly
                965                 970                 975

Glu Ala Lys Trp Gly Gln Thr Ser Lys Leu Ile Leu Leu Leu Pro His
            980                 985                 990

Gly Tyr Glu Gly Gln Gly Pro Asp His Ser Ser Ala Arg Ile Glu Arg
        995                 1000                1005

Phe Leu Gln Leu Cys Ala Glu Gly Ser Met Thr Val Ala Gln Pro Ser
    1010                1015                1020

Thr Pro Ala Asn His Phe His Leu Leu Arg Arg His Ala Leu Ser Asp
1025                1030                1035                1040

Leu Lys Arg Pro Leu Val Ile Phe Thr Pro Lys Ser Met Leu Arg Asn
                1045                1050                1055

Lys Ala Ala Ala Ser Ala Pro Glu Asp Phe Thr Glu Val Thr Lys Phe
            1060                1065                1070

Gln Ser Val Ile Asp Asp Pro Asn Val Ala Asp Ala Ala Lys Val Lys
        1075                1080                1085

Lys Val Met Leu Val Ser Gly Lys Leu Tyr Tyr Glu Leu Ala Lys Arg
    1090                1095                1100

Lys Glu Lys Asp Gly Arg Asp Asp Ile Ala Ile Val Arg Ile Glu Met
1105                1110                1115                1120

Leu His Pro Ile Pro Phe Asn Arg Ile Ser Glu Ala Leu Ala Gly Tyr
                1125                1130                1135

Pro Asn Ala Glu Glu Val Leu Phe Val Gln Asp Glu Pro Ala Asn Gln
            1140                1145                1150

Gly Pro Trp Pro Phe Tyr Gln Glu His Leu Pro Glu Leu Ile Pro Asn
        1155                1160                1165

Met Pro Lys Met Arg Arg Val Ser Arg Arg Ala Gln Ser Ser Thr Ala
    1170                1175                1180

Thr Gly Val Ala Lys Val His Gln Leu Glu Glu Lys Gln Leu Ile Asp
1185                1190                1195                1200

Glu Ala Phe Glu Ala
                1205


<210>    18
<211>    3618
<212>    DNA
<213>    Unknown

<220>
<223>    OdhA nucleotide sequence


<400>    18
atgttccagc agttccagaa ggaccccaag tccgtggaca aggaatggag agaactcttt        60

gaggcgcagg ggggaccaaa tgctaccccc gctacaacag aagcacagcc ttcagcgccc       120

aaggagtctg cgaaaccagc accaaaggct gcccctgcag ccaaggcagc accgcgcgta       180

gaaaccaagc cggccgccaa gaccgcccct aaggccaagg agtcctcagt gccacagcaa       240

cctaagcttc cggagccagg acaaacccca atcaggggta ttttcaagtc catcgcgaag       300

aacatggata tctccctgga aatcccaacc gcaacctcgg ttcgcgatat gccagctcgc       360

ctcatgttcg aaaaccgcgc gatggtcaac gatcagctca agcgcacccg cggtggcaag       420

atctccttca cccacatcat tggctacgcc atggtgaagg cagtcatggc tcacccggac       480

atgaacaact cctacgacgt catcgacggc aagccaaccc tgatcgtgcc tgagcacatc       540

aacctgggcc ttgccatcga ccttcctcag aaggacggct cccgcgcact tgtcgtagca       600

gccatcaagg aaaccgagaa gatgaacttc tccgagttcc tcgcagcata cgaagacatc 660

gtgacacgct cccgcaaggg caagctcacc atggatgact accagggcgt taccgtttcc 720

ttgaccaacc caggtggcat cggtacccgc cactctgtcc cacgtctgac caagggccag 780

ggcaccatca tcggtgtcgg ttccatggat tacccagcag agttccaggg cgcttccgaa 840

gaccgccttg cagagctcgg cgttggcaag cttgtcacca tcacctccac ctacgatcac 900

cgcgtgatcc agggtgctgt gtccggtgaa ttcctgcgta ccatgtctcg cctgctcacc 960

gatgattcct tctgggatga gatcttcgac gcaatgaacg ttccttacac cccaatgcgt 1020

tgggcacagg acgttccaaa caccggtgtt gataagaaca cccgcgtcat gcagctcatt 1080

gaggcatacc gctcccgtgg acacctcatc gctgacacca acccactttc atgggttcag 1140

cctggcatgc cagttccaga ccaccgcgac ctcgacatcg agacccacag cctgaccatc 1200

tgggatctgg accgtacctt cagcgtcggt ggcttcggcg gcaaggagac catgaccctg 1260

cgcgaggtac tgtcccgcct gcgcgctgcc tacaccttga aggtcggctc cgaatacacc 1320

cacatcctgg accgcgacga gcgcacctgg ctgcaggacc gcctcgaagc cggaatgcca 1380

aagccaaccc aggcagagca gaagtacatc ctgcagaagc tgaacgccgc agaggctttc 1440

gagaacttcc tgcagaccaa gtacgtcggc cagaagcgct tctccctcga aggtgcagaa 1500

gctctcatcc cactgatgga ctccgccatc gacaccgccg caggccaggg cctcgacgaa 1560

gttgtcatcg gtatgccaca ccgtggtcgc ctcaacgtgc tgttcaacat cgtgggcaag 1620

ccactggcat ccatcttcaa cgagtttgaa ggccaaatgg agcagggcca gatcggtggc 1680

tccggtgacg tgaagtacca cctcggttcc gaaggccagc acctgcagat gttcggcgac 1740

ggcgagatca aggtctccct gactgctaac ccgtcccacc tggaagctgt taacccagtg 1800

atggaaggta tcgtccgcgc aaagcaggac tacctggaca agggcgtaga cggcaagact 1860

gttgtgccac tgctgctcca cggtgacgct gcattcgcag gcctgggcat cgtgccagaa 1920

accatcaacc tggctaagct gcgtggctac gacgtcggag gcaccatcca catcgtggtg 1980

aacaaccaga tcggcttcac caccacccca gactccagcc gctccatgca ctacgcaacc 2040

gactacgcca aggcattcgg ctgcccagtc ttccacgtca atggtgatga cccagaggca 2100

gttgtctggg ttggccagct ggcaaccgag taccgtcgtc gcttcggcaa ggacgtcttc 2160

atcgacctcg tttgctaccg cctccgcggc cacaacgaag ctgatgatcc ttccatgacc 2220

cagccaaaga tgtatgagct catcaccggc cgcgagaccg ttcgtgctca gtacaccgaa 2280

gacctgctcg gacgtggaga cctctccaac gaagatgcag aagcagtcgt ccgcgacttc 2340

cacgaccaga tggaatctgt gttcaacgaa gtcaaggaag gcggcaagaa gcaggctgag 2400

gcacagaccg gcatcaccgg ctcccagaag cttccacacg gccttgagac caacatctcc 2460

cgtgaagagc tcctggaact gggacaggct ttcgccaaca ccccagaagg cttcaactac 2520

cacccacgtg tggctccagt tgctaagaag cgcgtctcct ctgtcaccga aggtggcatc 2580

gactgggcat ggggcgagct cctcgccttc ggttccctgg ctaactccgg ccgcttggtt 2640

cgccttgcag gtgaagattc ccgccgcggt accttcaccc agcgccacgc agttgccatc 2700

gacccagcga ccgctgaaga gttcaaccca ctccacgagc ttgcacagtc caagggcaac 2760

aacggtaagt tcctggtcta caactccgca ctgaccgagt acgcaggcat gggcttcgag 2820

tacggctact ccgtaggaaa cgaagactcc gtcgttgcat gggaagcaca gttcggcgac 2880

ttcgccaacg gcgctcagac catcatcgat gagtacgtct cctcaggcga agctaagtgg 2940

ggccagacct ccaagctgat ccttctgctg cctcacggct acgaaggcca gggcccagac 3000

cactcttccg cacgtatcga gcgcttcctg cagctgtgcg ctgagggttc catgactgtt 3060

gctcagccat ccaccccagc aaaccacttc cacctgctgc gtcgtcacgc tctgtccgac 3120

ctgaagcgtc cactggttat cttcaccccg aagtccatgc tgcgtaacaa ggctgctgcc 3180

tccgcaccag aagacttcac tgaggtcacc aagttccaat ccgtgatcga cgatccaaac 3240

gttgcagatg cagccaaggt gaagaaggtc atgctggtct ccggcaagct gtactacgaa 3300

ttggcaaagc gcaaggagaa ggacggacgc gacgacatcg cgatcgttcg tatcgaaatg 3360

ctccacccaa ttccgttcaa ccgcatctcc gaggctcttg ccggctaccc taacgctgag 3420

gaagtcctct tcgttcagga tgagccagca aaccagggcc catggccgtt ctaccaggag 3480

cacctcccag agctgatccc gaacatgcca aagatgcgcc gcgtttcccg ccgcgctcag 3540

tcctccaccg caactggtgt tgctaaggtg caccagctgg aggagaagca gcttatcgac 3600

gaggctttcg aggcttaa 3618

<210> 19
<211> 5803
<212> DNA
<213> Artificial Sequence

<220>
<223> pDCM2 nucleotide sequence

<400> 19
   gttcgcttgc tgtccataaa accgcccagt ctagctatcg ccatgtaagc ccactgcaag 60
   ctacctgctt tctctttgcg cttgcgtttt cccttgtcca gatagcccag tagctgacat 120
   tcatccgggg tcagcaccgt ttctgcggac tggctttcta cgtgttccgc ttcctttagc 180
   agcccttgcg ccctgagtgc ttgcggcagc gtgaagctag cttttatcgc cattcgccat 240
   tcaggctgcg caactgttgg gaagggcgat cggtgcgggc ctcttcgcta ttacgccagc 300
   tggcgaaagg gggatgtgct gcaaggcgat taagttgggt aacgccaggg ttttcccagt 360
   cacgacgttg taaaacgacg gccagtgaat tcgagctcgg tacccgggga tcctctagag 420
   tcgacctgca ggcatgcaag cttggcgtaa tcatggtcat agctgtttcc tgtgtgaaat 480
   tgttatccgc tcacaattcc acacaacata cgagccggaa gcataaagtg taaagcctgg 540
   ggtgcctaat gagtgagcta actcacatta attgcgttgc gctcactgcc cgctttccag 600
   tcgggaaacc tgtcgtgcca gctgcattaa tgaatcggcc aacgcgcggg gagaggcggt 660
   ttgcgtattg ggcgctcttc cgcttcctcg ctcactgact cgctgcgctc ggtcgttcgg 720
   ctgcggcgag cggtatcagc tcactcaaag gcggtaatac ggttatccac agaatcaggg 780
   gataacgcag gaaagaacat gtgagcaaaa ggccagcaaa aggccaggaa ccgtaaaaag 840
   gccgcgttgc tggcgttttt ccataggctc cgcccccctg acgagcatca caaaaatcga 900
   cgctcaagtc agaggtggcg aaacccgaca ggactataaa gataccaggc gtttccccct 960
   ggaagctccc tcgtgcgctc tcctgttccg accctgccgc ttaccggata cctgtccgcc 1020
   tttctccctt cgggaagcgt ggcgctttct caatgctcac gctgtaggta tctcagttcg 1080
   gtgtaggtcg ttcgctccaa gctgggctgt gtgcacgaac cccccgttca gcccgaccgc 1140
   tgcgccttat ccggtaacta tcgtcttgag tccaacccgg taagacacga cttatcgcca 1200
   ctggcagcag ccactggtaa caggattagc agagcgaggt atgtaggcgg tgctacagag 1260
   ttcttgaagt ggtggcctaa ctacggctac actagaagga cagtatttgg tatctgcgct 1320
   ctgctgaagc cagttacctt cggaaaaaga gttggtagct cttgatccgg caaacaaacc 1380
   accgctggta gcggtggttt ttttgtttgc aagcagcaga ttacgcgcag aaaaaaagga 1440
   tctcaagaag atcctttgat cttttctacg gggtctgacg ctcagtggaa cgaaaactca 1500
   cgttaaggga ttttggtcat gagattatca aaaaggatct tcacctagat ccttttgggg 1560
   tgggcgaaga actccagcat gagatccccg cgctggagga tcatccagcc ctgatagaaa 1620
   cagaagccac tggagcacct caaaaacacc atcatacact aaatcagtaa gttggcagca 1680
   tcacccgacg cactttgcgc cgaataaata cctgtgacgg aagatcactt cgcagaataa 1740
   ataaatcctg gtgtccctgt tgataccggg aagccctggg ccaacttttg gcgaaaatga 1800
   gacgttgatc ggcacgtaag aggttccaac tttcaccata atgaaataag atcactaccg 1860
   ggcgtatttt ttgagttatc gagattttca ggagctgata gaaacagaag ccactggagc 1920
   acctcaaaaa caccatcata cactaaatca gtaagttggc agcatcaccc gacgcacttt 1980
   gcgccgaata aatacctgtg acggaagatc acttcgcaga ataaataaat cctggtgtcc 2040
   ctgttgatac cgggaagccc tgggccaact tttggcgaaa atgagacgtt gatcggcacg 2100
   taagaggttc caactttcac cataatgaaa taagatcact accgggcgta ttttttgagt 2160
   tatcgagatt ttcaggagct ctttggcatc gtctctcgcc tgtcccctca gttcagtaat 2220
   ttcctgcatt tgcctgtttc cagtcggtag atattccaca aaacagcagg gaagcagcgc 2280
   ttttccgctg cataaccctg cttcggggtc attatagcga ttttttcggt atatccatcc 2340
   tttttcgcac gatatacagg attttgccaa agggttcgtg tagactttcc ttggtgtatc 2400
   caacggcgtc agccgggcag gataggtgaa gtaggcccac ccgcgagcgg gtgttccttc 2460
   ttcactgtcc cttattcgca cctggcggtg ctcaacggga atcctgctct gcgaggctgg 2520
   ccggctaccg ccggcgtaac agatgagggc aagcggatgg ctgatgaaac caagccaacc 2580
   aggaagggca gcccacctat caaggtgtac tgccttccag acgaacgaag agcgattgag 2640
   gaaaaggcgg cggcggccgg catgagcctg tcggcctacc tgctggccgt cggccagggc 2700
   tacaaaatca cgggcgtcgt ggactatgag cacgtccgcg agggcgtccc ggaaaacgat 2760
   tccgaagccc aacctttcat agaaggcggc ggtggaatcg aaatctcgtg atggcaggtt 2820
   gggcgtcgct tggtcggtca tttcgaaaaa ggttaggaat acggttagcc atttgcctgc 2880
   ttttatatag ttcantatgg gattcacctt tatgttgata agaaataaaa gaaaatgcca 2940
   ataggatatc ggcattttct tttgcgtttt tatttgttaa ctgttaattg tccttgttca 3000
   aggatgctgt ctttgacaac agatgttttc ttgcctttga tgttcagcag gaagctcggc 3060
   gcaaacgttg attgtttgtc tgcgtagaat cctctgtttg tcatatagct tgtaatcacg 3120
   acattgtttc ctttcgcttg aggtacagcg aagtgtgagt aagtaaaggt tacatcgtta 3180
   ggcggatcaa gatccatttt taacacaagg ccagttttgt tcagcggctt gtatgggcca 3240
   gttaaagaat tagaaacata accaagcatg taaatatcgt tagacgtaat gccgtcaatc 3300
   gtcatttttg atccgcggga gtcagtgaac aggtaccatt tgccgttcat tttaaagacg 3360
   ttcgcgcgtt caatttcatc tgttactgtg ttagatgcaa tcagcggttt catcactttt 3420
   ttcagtgtgt aatcatcgtt tagctcaatc ataccgagag cgccgtttgc taactcagcc 3480
   gtgcgttttt tatcgctttg cagaagtttt tgactttctt gacggaagaa tgatgtgctt 3540
   ttgccatagt atgctttgtt aaataaagat tcttcgcctt ggtagccatc ttcagttcca 3600
   gtgtttgctt caaatactaa gtatttgtgg cctttatctt ctacgtagtg aggatctctc 3660
   agcgtatggt tgtcgcctga gctgtagttg ccttcatcga tgaactgctg tacattttga 3720
   tacgtttttc cgtcaccgtc aaagattgat ttataatcct ctacaccgtt gatgttcaaa 3780
   gagctgtctg atgctgatac gttaacttgt gcagttgtca gtgtttgttt gccgtaatgt 3840
   ttaccggaga aatcagtgta gaataaacgg atttttccgt cagatgtaaa tgtggctgaa 3900
   cctgaccatt cttgtgtttg gtcttttagg atagaatcat ttgcatcgaa tttgtcgctg 3960
   tctttaaaga cgcggccagc gtttttccag ctgtcaatag aagtttcgcc gactttttga 4020
   tagaacatgt aaatcgatgt gtcatccgca tttttaggat ctccggctaa tgcaaagacg 4080
   atgtggtagc cgtgatagtt tgcgacagtg ccgtcagcgt tttgtaatgg ccagctgtcc 4140
   caaacgtcca ggccttttgc agaagagata tttttaattg tggacgaatc aaattcagaa 4200
   acttgatatt tttcattttt ttgctgttca gggatttgca gcatatcatg gcgtgtaata 4260
   tgggaaatgc cgtatgtttc cttatatggc ttttggttcg tttctttcgc aaacgcttga 4320
   gttgcgcctc ctgccagcag tgcggtagta aaggttaata ctgttgcttg ttttgcaaac 4380
   tttttgatgt tcatcgttca tgtctccttt tttatgtact gtgttagcgg tctgcttctt 4440
   ccagccctcc tgtttgaaga tggcaagtta gttacgcaca ataaaaaaag acctaaaata 4500
   tgtaaggggt gacgccaaag tatacacttt gccctttaca cattttaggt cttgcctgct 4560
   ttatcagtaa caaacccgcg cgatttactt ttcgacctca ttctattaga ctctcgtttg 4620
   gattgcaact ggtctatttt cctcttttgt ttgatagaaa atcataaaag gatttgcaga 4680
   ctacgggcct aaagaactaa aaaatctatc tgtttctttt cattctctgt attttttata 4740
   gtttctgttg catgggcata aagttgcctt tttaatcaca attcagaaaa tatcataata 4800
   tctcatttca ctaaataata gtgaacggca ggtatatgtg atgggttaaa aaggatcacc 4860
   ccagagtccc gctcagaaga actcgtcaag aaggcgatag aaggcgatgc gctgcgaatc 4920
   gggagcggcg ataccgtaaa gcacgaggaa gcggtcagcc cattcgccgc caagctcttc 4980
   agcaatatca cgggtagcca acgctatgtc ctgatagcgg tccgccacac ccagccggcc 5040
   acagtcgatg aatccagaaa agcggccatt ttccaccatg atattcggca agcaggcatc 5100
   gccatgggtc acgacgagat cctcgccgtc gggcatccgc gccttgagcc tggcgaacag 5160
   ttcggctggc gcgagcccct gatgctcttc gtccagatca tcctgatcga caagaccggc 5220
   ttccatccga gtacgtgctc gctcgatgcg atgtttcgct tggtggtcga atgggcaggt 5280
   agccggatca agcgtatgca gccgccgcat tgcatcagcc atgatggata ctttctcggc 5340
   aggagcaagg tgagatgaca ggagatcctg ccccggcact tcgcccaata gcagccagtc 5400
   ccttcccgct tcagtgacaa cgtcgagaca gctgcgcaag gaacgcccgt cgtggccagc 5460
   cacgatagcc gcgctgcctc gtcttggagt tcattcaggg caccggacag gtcggtcttg 5520
   acaaaaagaa ccgggcgccc ctgcgctgac agccggaaca cggcggcatc agagcagccg 5580
   attgtctgtt gtgcccagtc atagccgaat agcctctcca cccaagcggc cggagaacct 5640
   gcgtgcaatc catcttgttc aatcatgcga aacgatcctc atcctgtctc ttgatcagat 5700
   cttgatcccc tgcgccatca gatccttggc ggcaagaaag ccatccagtt tactttgcag 5760
   ggcttcccaa ccttaccaga gggcgcccca gctggcaatt ccg 5803

## Description

### [Technical Field]

The present disclosure relates to a novel glucosamine-6-phosphate deaminase variant, a *Corynebacterium glutamicum* strain comprising the variant, and a method for producing L-glutamic acid using the strain.

### [Background Art]

Various studies are being conducted to develop highly efficient microorganisms and fermentation process technologies for the production of L-amino acid and other useful substances. For example, a target substance-specific approach is mainly used in which the expression of a gene encoding an enzyme involved in L-glutamic acid biosynthesis is increased, or in which genes unnecessary for the biosynthesis are removed (US 8206954 B2).

However, it is still necessary to conduct studies to effectively increase the L-glutamic acid producing ability as the demand for L-glutamic acid increases.

### [Disclosure]

### [Technical Problem]

The present inventors have developed a novel glucosamine-6-phosphate deaminase variant, a *Corynebacterium glutamicum* strain comprising the variant, and a method for producing L-glutamic acid using the strain, thereby completing the present disclosure.

### [Technical Solution]

An object of the present disclosure is to provide a glucosamine-6-phosphate deaminase variant consisting of an amino acid sequence represented by SEQ ID NO: 1, in which alanine, which is an amino acid corresponding to position 137 of an amino acid sequence of SEQ ID NO: 3, is substituted with valine.

Another object of the present disclosure is to provide a polynucleotide encoding the variant of the present disclosure.

Still another object of the present disclosure is to provide a *Corynebacterium glutamicum* strain that comprises the variant of the present disclosure or a polynucleotide encoding the variant and has L-glutamic acid producing ability.

Still another object of the present disclosure is to provide a method for producing L-glutamic acid, which comprises culturing a *Corynebacterium glutamicum* strain that comprises a variant of the present disclosure or a polynucleotide encoding the variant and has L-glutamic acid producing ability in a medium.

### [Advantageous Effects]

In the case of culturing a *Corynebacterium glutamicum* strain comprising the glucosamine-6-phosphate deaminase variant of the present disclosure, it is possible to produce L-glutamic acid at a higher yield as compared to the case of existing microorganisms having unmodified polypeptides.

### [Description of Drawings]

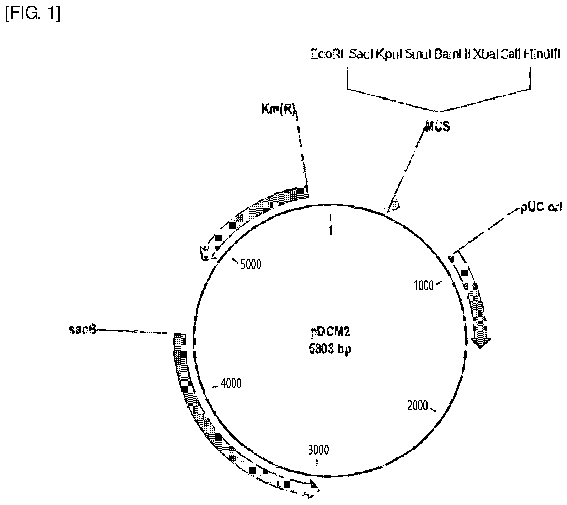

FIG. 1 is a schematic diagram of pDCM2 plasmid.

### [Best Mode]

The present disclosure will be described in detail as follows. Meanwhile, each of the descriptions and embodiments disclosed in the present disclosure may be applied to other descriptions and embodiments. In other words, all combinations of various elements disclosed in the present disclosure belong to the scope of the present disclosure. In addition, it cannot be considered that the scope of the present disclosure is limited by the specific description described below. In addition, throughout the present specification, a number of papers and patent documents are referenced and citations thereof indicated. The entirety of the content disclosed in the cited papers and patent documents is incorporated in the present specification by reference in order to more clearly describe the level of the technical field to which the present invention belongs and the content of the present invention.

An aspect of the present disclosure provides a glucosamine-6-phosphate deaminase variant consisting of an amino acid sequence represented by SEQ ID NO: 1, in which alanine, which is an amino acid corresponding to position 137 of an amino acid sequence of SEQ ID NO: 3, is substituted with valine.

The variant of the present disclosure may have, comprise, or consist essentially of the amino acid sequence represented by SEQ ID NO: 1.

In the variant of the present disclosure, the amino acid corresponding to position 137 based on the amino acid sequence of SEQ ID NO: 3 in the amino acid sequence represented by SEQ ID NO: 1 is valine, and may comprise an amino acid sequence having at least 70%, 75%, 80%, 85%, 90%, 95%, 96%, 97%, 98%, 99%, 99.5%, 99.7%, or 99.9% or more homology or identity to the amino acid sequence represented by SEQ ID NO: 1. It is apparent that variants having amino acid sequences in which some sequences are deleted, modified, substituted, conservatively substituted, or added are also included in the scope of the present disclosure as long as the amino acid sequences have such homology or identity and exhibit efficacy corresponding to that of the variant of the present disclosure.

Examples thereof include variants having sequence addition or deletion that do not alter the function of the variant of the present disclosure at the N-terminus, C-terminus of the amino acid sequence and/or inside the amino acid sequence, naturally occurring mutation, silent mutation, or conservative substitution.

The "conservative substitution" means the substitution of one amino acid with another amino acid having similar structural and/or chemical properties. Such an amino acid substitution may generally occur based on similarity in the polarity, charge, solubility, hydrophobicity, hydrophilicity, and/or amphipathic nature of the residues. Usually, conservative substitution may hardly affect or not affect the activity of the proteins or polypeptides.

In the present disclosure, the term "variant" refers to a polypeptide which has an amino acid sequence different from that of the variant before being varied by conservative substitution and/or modification of one or more amino acids but maintains the functions or properties. Such a variant may generally be identified by modifying one or more amino acids of the amino acid sequence of the polypeptide and evaluating the properties of the modified polypeptide. In other words, the ability of the variant may be increased, unchanged, or decreased as compared to that of the polypeptide before being varied. Some variants may include variants in which one or more portions such as an N-terminal leader sequence or a transmembrane domain have been removed. Other variants may include variants in which a portion of the N- and/or C-terminus has been removed from the mature protein. The term "variant" may be used interchangeably with terms such as modification, modified polypeptide, modified protein, variant, mutein, and divergent and is not limited thereto as long as it is a term used with the meaning of variation. For the purposes of the present disclosure, the variant may be a polypeptide comprising an amino acid sequence represented by SEQ ID NO: 1, in which alanine, which is an amino acid corresponding to position 137 of the amino acid sequence of SEQ ID NO: 3, is substituted with valine.

The variant may comprise deletions or additions of amino acids that have minimal effect on the properties and secondary structure of the polypeptide. For example, a signal (or leader) sequence that is cotranslationally or post-translationally involved in the protein translocation may be conjugated to the N-terminus of the variant. The variant may be conjugated with other sequences or linkers so as to be identified, purified, or synthesized.

In the present disclosure, the term "homology" or "identity" means the degree of similarity between two given amino acid sequences or base sequences and may be expressed as a percentage. The terms homology and identity may often be used interchangeably.

The sequence homology or identity of a conserved polynucleotide or polypeptide is determined by standard alignment algorithms, and the default gap penalty established by a program used may be used together. Substantially, homologous or identical sequences are generally capable of being hybridized with the entirety or a part of the sequence in moderately or highly stringent conditions. It is apparent that hybridization also includes hybridization of a polynucleotide with a polynucleotide comprising a general codon or a codon in consideration of codon degeneracy.

Whether any two polynucleotide or polypeptide sequences have homology, similarity, or identity may be determined using known computer algorithms such as the "FASTA" program, for example, using default parameters as in Pearson et al., (1988) Proc. Natl. Acad. Sci. USA 85:2444. Alternatively, the homology, similarity, or identity may be determined using Needleman-Wunsch algorithm (Needleman and Wunsch, 1970, J. Mol. Biol. 48:443-453) as performed in the Needleman program of the EMBOSS package (EMBOSS: The European Molecular Biology Open Software Suite, Rice et al., 2000, Trends Genet. 16:276-277) (version 5.0.0 or later) (including GCG program package (Devereux, J., et al., Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego,1994; and CARILLO et al. (1988) SIAM J Applied Math 48:1073). For example, BLAST of the National Center for Biotechnology Information or ClustalW may be used to determine the homology, similarity, or identity.

The homology, similarity, or identity of polynucleotides or polypeptides may be determined by comparing sequence information using, for example, a GAP computer program such as Needleman et al. (1970), J Mol Biol. 48:443 as announced in, for example, Smith and Waterman, Adv. Appl. Math (1981) 2:482. In summary, the GAP program may be defined as the value acquired by dividing the number of similarly aligned symbols (namely, nucleotides or amino acids) by the total number of symbols in the shorter of two sequences. The default parameters for the GAP program may include (1) a binary comparison matrix (including values of 1 for identity and 0 for non-identity) and a weighted comparison matrix of Gribskov et al., (1986) Nucl. Acids Res. 14:6745 (or EDNAFULL (EMBOSS version of NCBI NUC4.4) substitution matrix) as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

As an example of the present disclosure, the variant of the present disclosure may exhibit glucosamine-6-phosphate deaminase activity. The variant of the present disclosure may exhibit activity so as to have increased L-glutamic acid producing ability as compared to a wild-type polypeptide exhibiting glucosamine-6-phosphate deaminase activity. As used herein, the term "glucosamine-6-phosphate deaminase" refers to a polypeptide that converts acetyl glucosamine 6-phosphate into glucosamine 6-phosphate. Specifically, the term "glucosamine-6-phosphate deaminase" of the present disclosure may be used interchangeably with "glucosamine-6-phosphate isomerase", "GlcN6P deaminase", or "GNPDA". In the present disclosure, the sequence of the glucosamine-6-phosphate deaminase can be obtained from the GenBank of NCBI, a known database. Specifically, the glucosamine-6-phosphate deaminase may be a polypeptide having glucosamine-6-phosphate deaminase activity encoded by the *nagB* gene, but is not limited thereto.

In the present disclosure, the term "corresponding to" refers to amino acid residues at positions listed in the polypeptide or amino acid residues that are similar, identical, or homologous to those listed in the polypeptide. Identifying the amino acid at the corresponding position may be determining a specific amino acid in a sequence that refers to a specific sequence. As used herein, "corresponding region" generally refers to a similar or corresponding position in a related protein or a reference protein.

For example, an arbitrary amino acid sequence is aligned with SEQ ID NO: 3, and based on this, each amino acid residue of the amino acid sequence may be numbered with reference to the amino acid residue of SEQ ID NO: 3 and the numerical position of the corresponding amino acid residue. For example, a sequence alignment algorithm as described in the present disclosure may determine the position of an amino acid or a position at which modification such as substitution, insertion, or deletion occurs through comparison with that in a query sequence (also referred to as a "reference sequence").

For such alignments, for example, the Needleman-Wunsch algorithm (Needleman and Wunsch, 1970, J. Mol. Biol. 48:443-453), the Needleman program of the EMBOSS package (EMBOSS: The European Molecular Biology Open Software Suite, Rice et al., 2000, Trends Genet. 16:276-277), and the like may be used, but the program and algorithm are not limited thereto, and a sequence alignment program, a pairwise sequence comparison algorithm, and the like known in the art may be appropriately used.

Another aspect of the present disclosure is to provide a polynucleotide encoding the variant of the present disclosure.

In the present disclosure, the term "polynucleotide" is a DNA or RNA strand having a certain length or more as a polymer of nucleotides in which nucleotide monomers are connected in a long chain by covalent bonds, and more specifically means a polynucleotide fragment encoding the variant.

The polynucleotide encoding the variant of the present disclosure may comprise a base sequence encoding the amino acid sequence represented by SEQ ID NO: 1. As an example of the present disclosure, the polynucleotide of the present disclosure may have or comprise the sequence of SEQ ID NO: 2. The polynucleotide of the present disclosure may consist of or consist essentially of the sequence of SEQ ID NO: 2.

In the polynucleotide of the present disclosure, various modifications may be made in the coding region as long as the amino acid sequence of the variant of the present disclosure is not changed in consideration of codon degeneracy or codons preferred in organisms that are intended to express the variant of the present disclosure. Specifically, the polynucleotide of the present disclosure has or comprises a base sequence having 70% or more, 75% or more, 80% or more, 85% or more, 90% or more, 95% or more, 96% or more, 97% or more, 98% or more, but less than 100% homology or identity to the sequence of SEQ ID NO: 2 or may consist of or consist essentially of a base sequence having 70% or more, 75% or more, 80% or more, 85% or more, 90% or more, 95% or more, 96% or more, 97% or more, 98% or more, but less than 100% homology or identity to the sequence of SEQ ID NO: 2, but is not limited thereto. Here, in the sequence having homology or identity, the codon encoding the amino acid corresponding to position 137 of SEQ ID NO: 1 may be one of the codons encoding valine.

The polynucleotide of the present disclosure may comprise a probe that can be prepared from a known gene sequence, for example, a sequence without limitation as long as it is a sequence that can hybridize with a complementary sequence to the entirety or a part of the polynucleotide sequence of the present disclosure under stringent conditions. The "stringent conditions" mean conditions that enable specific hybridization between polynucleotides. These conditions are specifically described in documents (see J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). Examples thereof include a condition in which polynucleotides having higher homology or identity, namely polynucleotides having 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed one time, specifically two to three times at a salt concentration and temperature equivalent to 60°C, 1X SSC, 0.1% SDS, specifically 60°C, 0.1X SSC, 0.1% SDS, more specifically 68°C, 0.1X SSC, 0.1% SDS, which are washing conditions for common Southern hybridization.

Hybridization requires that two nucleic acids have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

Specifically, a polynucleotide having homology or identity to the polynucleotide of the present disclosure may be detected using a hybridization condition including a hybridization step at a Tₘ value of 55°C and the above-described conditions. The Tₘ value may be 60°C, 63°C, or 65°C, but is not limited thereto, and may be appropriately adjusted by those skilled in the art according to the purpose.

The appropriate stringency to hybridize the polynucleotide depends on the length and degree of complementarity of the polynucleotide, and the variables are well known in the art (for example, J. Sambrook et al., Molecular Cloning, A Laboratory Manual, 2nd Edition, Cold Spring Harbor Laboratory press, Cold Spring Harbor, New York, 1989).

Another aspect of the present disclosure is to provide a vector comprising the polynucleotide of the present disclosure. The vector may be an expression vector for expressing the polynucleotide in a host cell, but is not limited thereto.

The vector of the present disclosure may include a DNA construct comprising a polynucleotide sequence encoding a polypeptide of interest operably linked to a suitable expression control region (or expression control sequence) so that the polypeptide of interest may be expressed in a suitable host. The expression control region may contain a promoter capable of initiating transcription, any operator sequence for controlling the transcription, a sequence encoding a suitable mRNA ribosome binding site, and a sequence controlling termination of transcription and translation. The vector may be transformed into a suitable host cell and then replicated or function independently of the host genome, or may be integrated into the genome itself.

The vector used in the present disclosure is not particularly limited, but any vector known in the art may be used. Examples of commonly used vectors include natural or recombinant plasmids, cosmids, viruses, and bacteriophages. For example, pWE15, M13, MBL3, MBL4, IXII, ASHII, APII, t10, t11, Charon4A, Charon21A, and the like may be used as a phage vector or a cosmid vector, and pDZ system, pBR system, pUC system, pBluescript II system, pGEM system, pTZ system, pCL system, pET system, and the like may be used as a plasmid vector. Specifically, pDZ, pDC, pDCM2, pACYC177, pACYC184, pCL, pECCG117, pUC19, pBR322, pMW118, and pCC1BAC vectors and the like may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further contain a selection marker for the confirmation of chromosome insertion. The selection marker is for selecting the cells transformed with vectors, that is, for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells can be selected.

In the present disclosure, the term "transformation" means that a vector comprising a polynucleotide encoding a target polypeptide is introduced into a host cell or a microorganism so that the polypeptide encoded by the polynucleotide can be expressed in the host cell. The transformed polynucleotide may be located by being inserted into the chromosome of the host cell or located outside the chromosome as long as it can be expressed in the host cell. The polynucleotide comprises DNA and/or RNA encoding a polypeptide of interest. The polynucleotide may be introduced in any form as long as it can be introduced into a host cell and expressed. For example, the polynucleotide may be introduced into a host cell in the form of an expression cassette, which is a gene construct containing all elements required for selfexpression. The expression cassette may usually contain a promoter operably linked to the polynucleotide, a transcription termination signal, a ribosome binding site, and a translation termination signal. The expression cassette may be in the form of an expression vector capable of self-replicating. The polynucleotide may be introduced into a host cell in its own form and operably linked to a sequence required for expression in the host cell, but is not limited thereto.

In the above, the term "operably linked" means that the polynucleotide sequence is functionally linked to a promoter sequence that initiates and mediates transcription of the polynucleotide encoding the variant of interest of the present disclosure.

Still another aspect of the present disclosure is to provide a *Corynebacterium glutamicum* strain that comprises the variant of the present disclosure or the polynucleotide of the present disclosure.

The strain of the present disclosure may comprise a modified polypeptide of the present disclosure, a polynucleotide encoding the polypeptide, or a vector comprising the polynucleotide of the present disclosure.

In the present disclosure, the "strain (or microorganism)" includes all wild-type microorganisms or naturally or artificially genetically modified microorganisms, and it may be a microorganism in which a specific mechanism is weakened or strengthened due to a insertion of an external gene or an activity enhancement or inactivation of an endogenous gene, and it may be a microorganism comprising genetic modification for production of a polypeptide, protein, or product of interest.

The strain of the present disclosure may be a strain comprising any one or more of the variant of the present disclosure, the polynucleotide of the present disclosure, or a vector comprising the polynucleotide of the present disclosure; a strain modified to express the variant of the present disclosure or the polynucleotide of the present disclosure; a strain (for example, a recombinant strain) expressing the variant of the present disclosure or the polynucleotide of the present disclosure; or a strain (for example, a recombinant strain) exhibiting the activity of the variant of the present disclosure, but is not limited thereto.

The strain of the present disclosure may be a strain having L-glutamic acid producing ability.

The strain of the present disclosure may be a microorganism naturally having glucosamine-6-phosphate deaminase or L-glutamic acid producing ability or a microorganism in which the variant of the present disclosure or a polynucleotide encoding the variant (or a vector comprising the polynucleotide) is introduced into a parent strain that does not have glucosamine-6-phosphate deaminase or L-glutamic acid producing ability and/or in which L-glutamic acid producing ability is conferred on the parent strain, but is not limited thereto.

For example, the strain of the present disclosure is a cell or microorganism that is transformed with a vector comprising the polynucleotide of the present disclosure or a polynucleotide encoding the variant of the present disclosure and expresses the variant of the present disclosure. For the purposes of the present disclosure, the strain of the present disclosure may include all microorganisms that comprise the variant of the present disclosure and can produce L-glutamic acid. For example, the strain of the present disclosure may be a recombinant strain in which a polynucleotide encoding the variant of the present disclosure is introduced into a natural wild-type microorganism or a microorganism producing L-glutamic acid to thus express a glucosamine-6-phosphate deaminase variant and having increased L-glutamic acid producing ability. The recombinant strain having increased L-glutamic acid producing ability may be a microorganism having increased L-glutamic acid producing ability as compared to a natural wild-type microorganism or a glucosamine-6-phosphate deaminase unmodified microorganism (namely, a microorganism expressing wild-type glucosamine-6-phosphate deaminase (SEQ ID NO: 3) or a microorganism that does not express modified (SEQ ID NO: 1) protein), but is not limited thereto. For example, the strain having increased L-glutamic acid producing ability of the present disclosure may be a microorganism having increased L-glutamic acid producing ability as compared to *Corynebacterium glutamicum* which comprises a polypeptide of SEQ ID NO: 3 or a polynucleotide encoding the polypeptide, but is not limited thereto. As an example, the glucosamine-6-phosphate deaminase unmodified microorganism that is a target strain for comparison of the increase in L-glutamic acid producing ability may be an ATCC13869 or ATCC13869 ΔodhA strain (Appl Environ Microbiol. 2007 Feb;73(4):1308-19. Epub 2006 Dec 8.), but is not limited thereto.

For example, the recombinant strain having increased producing ability may have L-glutamic acid producing ability increased by about 1% or more, specifically about 1% or more, about 2.5% or more, about 5% or more, about 6% or more, about 7% or more, about 8% or more, about 9% or more, about 10% or more, about 10.5% or more, about 11% or more, about 11.5% or more, about 12% or more, about 12.5% or more, about 13% or more, about 13.5% or more, about 14% or more, about 14.5% or more, about 15% or more, about 15.5% or more, about 16% or more, about 16.5% or more, about 17% or more, about 17.5% or more, about 18% or more, about 18.5% or more, about 19% or more, about 19.5% or more, about 20% or more, about 20.5% or more, about 21% or more, about 21.5% or more, about 22% or more, about 22.5% or more, about 23% or more, about 23.5% or more, about 24% or more, or about 24.5% or more, or about 25% or more, or about 25.5% or more, or about 26% or more, or about 26.5% or more, or about 27% or more, or about 27.5% or more, or about 28% or more, or about 28.5% or more, or about 29% or more, or about 29.5% or more, or about 30% or more, or about 30.5% or more, or about 31% or more, or about 31.5% or more, or about 32% or more, or about 32.5% or more, or about 33% or more, or about 33.5% or more, or about 34% or more, or about 34.5% or more, or about 35% or more, or about 35.5% or more, or about 36% or more, or about 36.5% or more (the upper limit is not particularly limited and may be, for example, about 200% or less, about 150% or less, about 100% or less, about 50% or less, about 45% or less, about 40% or less, about 37% or less) as compared to the L-glutamic acid producing ability of the parent strain before being varied or an unmodified microorganism, but the increased amount is not limited thereto as long as the producing ability has an increased amount of a + value as compared to the producing ability of the parent strain before being varied or an unmodified microorganism. In another example, the recombinant strain having increased producing ability may have L-glutamic acid producing ability increased by about 1.1 times or more, about 1.12 times or more, about 1.13 times or more, about 1.15 times or more, about 1.16 times or more, about 1.17 times or more, about 1.18 times or more, about 1.19 times or more, about 1.2 times or more, or about 1.25 times or more, about 1.3 times or more, about 1.35 times or more, about 1.36 times or more (the upper limit is not particularly limited and may be, for example, about 10 times or less, about 5 times or less, about 3 times or less, or about 2 times or less) as compared to the L-glutamic acid producing ability of the parent strain before being varied or an unmodified microorganism, but the rate of increase is not limited thereto. The term "about" is a range including all of ±0.5, ±0.4, ±0.3, ±0.2, ±0.1, and the like, includes all values in a range equal to or similar to the value following the term "about", but is not limited thereto.

In the present disclosure, the "unmodified microorganism" does not exclude strains comprising mutation that may occur naturally in microorganisms, and may be a wild-type strain or a natural strain itself or may be a strain before the trait is changed by genetic variation due to natural or artificial factors. For example, the unmodified microorganism may be a strain into which the glucosamine-6-phosphate deaminase variant described in the present specification is not introduced or has not yet been introduced. The term "unmodified microorganism" may be used interchangeably with "strain before being modified", "microorganism before being modified", "unvaried strain", "unmodified strain", "unvaried microorganism", or "reference microorganism".

In another example of the present disclosure, the microorganism of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

The microorganism of the present disclosure may be a microorganism in which the activity of OdhA protein is further weakened or inactivated.

In the present disclosure, the "weakening" of a polypeptide activity includes both cases where the activity is decreased as compared to the endogenous activity or absent. The weakening may be used interchangeably with terms such as inactivation, deficiency, down-regulation, decrease, reduce, and attenuation.

The weakening may also include a case where the activity of the polypeptide itself is decreased or eliminated as compared to the activity of the polypeptide originally possessed by the microorganism by variation of the polynucleotide encoding the polypeptide, and the like, a case where the overall polypeptide activity level and/or concentration (expression level) in the cell is lower as compared to that of the natural strain by inhibition of the expression of the gene of the polynucleotide encoding the polypeptide or inhibition of translation into the polypeptide, a case where the polynucleotide is not expressed at all, and/or a case where the polypeptide activity is not exhibited even when the polynucleotide is expressed. The "endogenous activity" means the activity of a specific polypeptide originally possessed by the parent strain before the trait is changed or a wild-type or unmodified microorganism when the trait is changed by genetic variation due to natural or artificial factors. The endogenous activity may be used interchangeably with "activity before modification". The fact that the activity of a polypeptide is "inactivated, deficient, decreased, down-regulated, reduced, or attenuated" as compared to the endogenous activity means that the activity of a polypeptide is lowered as compared to the activity of a specific polypeptide originally possessed by the parent strain before the trait is changed or a wild-type or unmodified microorganism.

Such weakening of the activity of a polypeptide may be performed by any method known in the art, but the method is not limited thereto, and the weakening may be achieved by applying various methods well known in the art (for example, Nakashima N. et al., Bacterial cellular engineering by genome editing and gene silencing. Int J Mol Sci. 2014; 15(2):2773-2793, Sambrook et al., Molecular Cloning 2012, and the like).

Specifically, the weakening of the polypeptide activity in the present disclosure may be:
1) deletion of the entirety or a part of a gene encoding a polypeptide;
2) modification of an expression control region (or expression control sequence) to decrease the expression of a gene encoding a polypeptide;
3) modification of an amino acid sequence constituting the polypeptide to eliminate or weaken the activity of the polypeptide (for example, deletion/substitution/addition of one or more amino acids in an amino acid sequence);
4) modification of a gene sequence encoding a polypeptide to eliminate or weaken the activity of the polypeptide (for example, deletion/substitution/addition of one or more nucleic acid bases in a nucleic acid base sequence of the polypeptide gene to encode a polypeptide that has been modified to eliminate or weaken the activity of the polypeptide);
5) modification of a start codon of a gene transcript encoding a polypeptide or a base sequence encoding a 5'-UTR region;
6) introduction of an antisense oligonucleotide (for example, antisense RNA) that complementarily binds to the transcript of the gene encoding a polypeptide;
7) addition of a sequence complementary to a Shine-Dalgarno sequence in front of the Shine-Dalgarno sequence of a gene encoding a polypeptide in order to form a secondary structure to which ribosome cannot be attached;
8) addition of a promoter to be transcribed in the opposite direction to the 3' end of the open reading frame (ORF) of a gene sequence encoding a polypeptide (reverse transcription engineering, RTE); or
9) a combination of two or more selected from 1) to 8), but is not particularly limited thereto.

For example:
1) The deletion of a part or the entirety of the gene encoding a polypeptide may be removal of the entire polynucleotide encoding the endogenous polypeptide of interest in the chromosome, replacement with a polynucleotide in which some nucleotides are deleted or replacement with a marker gene.
2) The modification of the expression control region (or expression control sequence) may be deletion, insertion, non-conservative or conservative substitution, or occurrence of variation in the expression control region (or expression control sequence) due to a combination thereof or replacement with a sequence exhibiting weaker activity. The expression control region contains a promoter, an operator sequence, a sequence encoding a ribosome binding site, and a sequence controlling the termination of transcription and translation, but is not limited thereto.
5) The modification of a start codon of a gene transcript encoding a polypeptide or a base sequence encoding a 5'-UTR region may be, for example, substitution with a base sequence encoding another start codon having a lower polypeptide expression rate as compared to the endogenous start codon, but is not limited thereto.
3) and 4) The modification of an amino acid sequence or polynucleotide sequence may be deletion, insertion, or non-conservative or conservative substitution of an amino acid sequence of the polypeptide or a polynucleotide sequence encoding the polypeptide or occurrence of variation in the sequence due to a combination thereof or replacement with an amino acid sequence or polynucleotide sequence modified to exhibit weaker activity or an amino acid sequence or polynucleotide sequence modified to be inactive so that the activity of the polypeptide is weakened, but is not limited thereto. For example, the expression of a gene may be inhibited or weakened by introducing variation into the polynucleotide sequence and forming a stop codon, but the modification is not limited thereto.
6) For the introduction of an antisense oligonucleotide (for example, antisense RNA) that complementarily binds to the transcript of the gene encoding a polypeptide, reference may be made to documents, for example, Weintraub, H. et al., Antisense-RNA as a molecular tool for genetic analysis, Reviews-Trends in Genetics, Vol. 1(1) 1986.
7) The addition of a sequence complementary to a Shine-Dalgarno sequence in front of the Shine-Dalgarno sequence of a gene encoding a polypeptide in order to form a secondary structure to which ribosome cannot be attached may be to make mRNA translation impossible or to slow down the mRNA translation rate.
8) The addition of a promoter to be transcribed in the opposite direction to the 3' end of the open reading frame (ORF) of a gene sequence encoding a polypeptide (reverse transcription engineering, RTE) may be to weaken the activity by making an antisense nucleotide complementary to the transcript of the gene encoding a polypeptide.

In the present disclosure, the term "enhancement" of polypeptide activity means that the activity of a polypeptide is increased as compared to the endogenous activity. The enhancement may be used interchangeably with terms such as activation, up-regulation, overexpression, and increase. Here, activation, enhancement, up-regulation, overexpression, and increase may include both exhibiting activity that was not originally possessed and exhibiting improved activity as compared to the endogenous activity or activity before modification. The "endogenous activity" means the activity of a specific polypeptide originally possessed by the parent strain before the trait is changed or an unmodified microorganism when the trait is changed by genetic variation due to natural or artificial factors. This may be used interchangeably with "activity before modification". The fact that the activity of a polypeptide is "enhanced", "up-regulated", "overexpressed", or "increased" as compared to the endogenous activity means that the activity of a polypeptide is improved as compared to the activity and/or concentration (expression level) of a specific polypeptide originally possessed by the parent strain before the trait is changed or an unmodified microorganism.

The enhancement may be achieved through the introduction of a foreign polypeptide or the enhancement of endogenous activity and/or concentration (expression level) of the polypeptide. The enhancement of the activity of a polypeptide may be confirmed by an increase in the degree of activity and the expression level of the polypeptide or in the amount of the product produced from the polypeptide.

For the enhancement of the activity of a polypeptide, various methods well known in the art may be applied, and the method is not limited as long as the activity of the polypeptide of interest can be enhanced as compared to that of the microorganism before being modified. Specifically, genetic engineering and/or protein engineering well known to those skilled in the art, which are routine methods of molecular biology, may be used, but the method is not limited thereto (for example, Sitnicka et al., Functional Analysis of Genes. Advances in Cell Biology. 2010, Vol. 2. 1-16; Sambrook et al., Molecular Cloning 2012; and the like).

Specifically, the enhancement of the activity of a polypeptide of the present disclosure may be:
1) increase in the intracellular copy number of a polynucleotide encoding a polypeptide;
2) replacement of a gene expression regulatory region on a chromosome encoding a polypeptide with a sequence exhibiting strong activity;
3) modification of a start codon of a gene transcript encoding a polypeptide or a base sequence encoding a 5'-UTR region;
4) modification of an amino acid sequence of a polypeptide to enhance the activity of the polypeptide;
5) modification of a polynucleotide sequence encoding a polypeptide to enhance the activity of the polypeptide (for example, modification of a polynucleotide sequence of the polypeptide gene to encode a polypeptide that has been modified to enhance the activity of the polypeptide);
6) introduction of a foreign polypeptide exhibiting the activity of a polypeptide or a foreign polynucleotide encoding the polypeptide;
7) codon optimization of a polynucleotide encoding a polypeptide;
8) analysis of the tertiary structure of a polypeptide to select and modification or chemical modification of the exposed site; or
9) a combination of two or more selected from 1) to 8), but is not particularly limited thereto.

More specifically:
1) The increase in the intracellular copy number of a polynucleotide encoding a polypeptide may be achieved by the introduction into a host cell of a vector which can be replicated and function independently of the host and to which a polynucleotide encoding the polypeptide is operably linked. Alternatively, the increase may be achieved by the introduction of one copy or two or more copies of a polynucleotide encoding the polypeptide into a chromosome of a host cell. The introduction into the chromosome may be performed by introducing a vector capable of inserting the polynucleotide into a chromosome of a host cell into the host cell, but is not limited thereto. The vector is as described above.
2) The replacement of a gene expression control region (or expression control sequence) on a chromosome encoding a polypeptide with a sequence exhibiting strong activity may be, for example, deletion, insertion, non-conservative or conservative substitution, or occurrence of variation in a sequence due to a combination thereof or replacement with a sequence exhibiting stronger activity so that the activity of the expression control region is further enhanced. The expression control region is not particularly limited thereto, but may contain a promoter, an operator sequence, a sequence encoding a ribosome binding site, a sequence controlling the termination of transcription and translation, and the like. For example, the replacement may be to replace the original promoter with a strong promoter, but is not limited thereto.

Examples of known strong promoters include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, and yccA promoter, but are not limited thereto.
3) The modification of a start codon of a gene transcript encoding a polypeptide or a base sequence encoding a 5'-UTR region may be, for example, substitution with a base sequence encoding another start codon having a higher polypeptide expression rate as compared to an endogenous start codon, but is not limited thereto.
4) and 5) The modification of an amino acid sequence or polynucleotide sequence may be deletion, insertion, non-conservative or conservative substitution of an amino acid sequence of the polypeptide or a polynucleotide sequence encoding the polypeptide or occurrence of variation in the sequence due to a combination thereof or replacement with an amino acid sequence or polynucleotide sequence modified to exhibit stronger activity or an amino acid sequence or polynucleotide sequence modified to be more active so that the activity of the polypeptide is enhanced, but is not limited thereto. The replacement may be specifically performed by inserting a polynucleotide into a chromosome by homologous recombination, but is not limited thereto. The vector used here may further contain a selection marker for the confirmation of chromosome insertion. The selection marker is as described above.
6) The introduction of a foreign polypeptide exhibiting the activity of a polypeptide may be the introduction of a foreign polynucleotide encoding a polypeptide exhibiting activity the same as or similar to that of the polypeptide into a host cell. The foreign polynucleotide is not limited in its origin or sequence as long as it exhibits activity the same as or similar to that of the polypeptide. The introduction may be performed by appropriately selecting a known transformation method by those skilled in the art. As the introduced polynucleotide is expressed in a host cell, a polypeptide may be produced, and the activity thereof may be increased.
7) The codon optimization of a polynucleotide encoding a polypeptide may be codon optimization of an endogenous polynucleotide so as to increase transcription or translation in a host cell or codon optimization of a foreign polynucleotide so as to perform optimized transcription and translation in a host cell.
8) The analysis of the tertiary structure of a polypeptide to select and modification or chemical modification of the exposed site may be, for example, to determine a template protein candidate according to the degree of similarity of the sequence by comparing the sequence information of a polypeptide to be analyzed with a database storing the sequence information of known proteins, to confirm the structure based on this, and to select and modify or chemically modify the exposed portion to be modified or chemically modified.

Such enhancement of the polypeptide activity may be an increase in the activity or concentration expression level of the corresponding polypeptide based on the activity or concentration of the polypeptide expressed in a wild-type microbial strain or a microbial strain before being modified or an increase in the amount of a product produced from the polypeptide, but is not limited thereto.

More specifically, the *Corynebacterium glutamicum* strain of the present disclosure may be a microorganism in which a polypeptide comprising SEQ ID NO: 17, a nucleotide encoding the polypeptide comprising SEQ ID NO: 17, or a nucleotide comprising SEQ ID NO: 18 is further deleted.

In the microorganism of the present disclosure, partial or entire modification of a polynucleotide may be induced by (a) homologous recombination using a vector for chromosome insertion in the microorganism or genome editing using engineered nuclease (e.g., CRISPR-Cas9) and/or (b) treatment with light such as ultraviolet rays and radiation and/or chemicals, but is not limited thereto. A method for modifying a part or the entirety of the gene may include a method using DNA recombination technology. For example, by introducing a nucleotide sequence or vector containing a nucleotide sequence homologous to the gene of interest into the microorganism to cause homologous recombination, a part or the entirety of the gene may be deleted. The introduced nucleotide sequence or vector may contain a dominant selection marker, but is not limited thereto.

In the microorganism of the present disclosure, the variant, polynucleotide, L-glutamic acid, and the like are as described in the other aspects.

Still another aspect of the present disclosure provides a method for producing L-glutamic acid, which includes culturing a *Corynebacterium glutamicum* strain comprising the variant of the present disclosure or the polynucleotide of the present disclosure in a medium.

The method for producing L-glutamic acid of the present disclosure may include culturing a *Corynebacterium glutamicum* strain comprising the variant of the present disclosure or the polynucleotide of the present disclosure or the vector of the present disclosure in a medium.

In the present disclosure, the term "culture" means growing the *Corynebacterium glutamicum* strain of the present disclosure under appropriately controlled environmental conditions. The culture process of the present disclosure may be performed according to suitable medium and culture conditions known in the art. Such a culture process may be easily adjusted and used by those skilled in the art according to the selected strain. Specifically, the culture may be a batch type, continuous type, and/or fed-batch type, but is not limited thereto.

In the present disclosure, the term "medium" means a mixed substance containing nutrients required to culture the *Corynebacterium glutamicum* strain of the present disclosure as a main component, and the medium supplies nutrients, growth factors, and the like including water that are indispensable for survival and development. Specifically, as the medium and other culture conditions used for culture of the *Corynebacterium glutamicum* strain of the present disclosure, any one may be used without particular limitation as long as it is a medium used for common culture of microorganisms. The *Corynebacterium glutamicum* strain of the present disclosure may be cultured in a common medium containing proper carbon sources, nitrogen sources, phosphorus sources, inorganic compounds, amino acids and/or vitamins, and the like while controlling the temperature, pH, and the like under aerobic conditions.

Specifically, the culture medium for the strain of the genus *Corynebacterium* may be found in the document "Manual of Methods for General Bacteriology" by the American Society for Bacteriology (Washington, D.C., USA, 1981).

In the present disclosure, the carbon sources include carbohydrates such as glucose, saccharose, lactose, fructose, sucrose, and maltose; sugar alcohols such as mannitol and sorbitol, organic acids such as pyruvic acid, lactic acid, and citric acid; amino acids such as glutamic acid, methionine, and lysine; and the like. Natural organic nutrients such as starch hydrolysate, molasses, blackstrap molasses, rice bran, cassava, sugarcane residue, and corn steep liquor may be used. Specifically, carbohydrates such as glucose and sterilized pretreated molasses (namely, molasses converted to reducing sugar) may be used, and appropriate amounts of other carbon sources may be used in various manners without limitation. These carbon sources may be used singly or in a combination of two or more, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate; and organic nitrogen sources such as amino acids such as glutamic acid, methionine, and glutamine, peptone, NZ-amine, meat extract, yeast extract, malt extract, corn steep liquor, casein hydrolysate, fish or decomposition products thereof, and skim soybean cake or decomposition products thereof may be used. These nitrogen sources may be used singly or in a combination of two or more, but are not limited thereto.

The phosphorus sources may include monopotassium phosphate, dipotassium phosphate, or sodium-containing salts corresponding thereto. As the inorganic compounds, sodium chloride, calcium chloride, iron chloride, magnesium sulfate, iron sulfate, manganese sulfate, calcium carbonate, and the like may be used. In addition to these, amino acids, vitamins and/or suitable precursors, and the like may be contained. These components or precursors may be added to the medium batchwise or continuously, but the manner of addition is not limited thereto.

During the culture of the *Corynebacterium glutamicum* strain of the present disclosure, the pH of the medium may be adjusted by adding compounds such as ammonium hydroxide, potassium hydroxide, ammonia, phosphoric acid, and sulfuric acid to the medium in a proper manner. During the culture, foaming may be suppressed by using an antifoaming agent such as fatty acid polyglycol ester. Oxygen or oxygen-containing gas may be injected into the medium in order to maintain the aerobic state of the medium, or gas may not be injected or nitrogen, hydrogen, or carbon dioxide gas may be injected in order to maintain the anaerobic and microaerobic states, but the method for maintaining the state is not limited thereto.

In the culture of the present disclosure, the culture temperature may be maintained at 20°C to 45°C, specifically 25°C to 40°C, and the strain may be cultured for about 10 to 160 hours, but the culture conditions are not limited thereto.

L-glutamic acid produced through the culture of the present disclosure may be secreted into the medium or may remain in the cells.

The method for producing L-glutamic acid of the present disclosure may further comprise a step of preparing the *Corynebacterium glutamicum* strain of the present disclosure, a step of preparing a medium for culture of the strain, or a combination of these (in any order), for example, prior to the culture step.

The method for producing L-glutamic acid of the present disclosure may further include a step of recovering L-glutamic acid from the medium according to the culture (the medium subjected to the culture) or from the *Corynebacterium glutamicum* strain. The recovery step may be further included after the culture step.

The recovery may be to collect the L-glutamic acid of interest by way of a suitable method known in the art according to the method for culturing the microorganism of the present disclosure, for example, a batch, continuous, or fed-batch culture method. For example, centrifugation, filtration, treatment with a crystallized protein precipitant (salting out), extraction, ultrasonic disintegration, ultrafiltration, dialysis, various forms of chromatography such as molecular-sieve chromatography (gel filtration), adsorption chromatography, ion-exchange chromatography, and affinity chromatography, HPLC, or a combination thereof may be used. The L-glutamic acid of interest may be recovered from the medium or microorganism by way of a suitable method known in the art.

The method for producing L-glutamic acid of the present disclosure may further include a purification step. The purification may be performed by way of a suitable method known in the art. In an example, when the method for producing L-glutamic acid of the present disclosure includes both the recovery step and the purification step, the recovery step and the purification step may be performed continuously or discontinuously regardless of the order, or may be performed simultaneously or by being combined into one step, but the manner to perform the steps is not limited thereto.

In the method of the present disclosure, the variant, polynucleotide, vector, strain, L-glutamic acid, and the like are as described in the other aspects.

Still another aspect of the present disclosure is to provide a composition for L-glutamic acid production, which comprises a *Corynebacterium glutamicum* strain comprising the variant of the present disclosure, a polynucleotide encoding the variant, a vector comprising the polynucleotide, or the polynucleotide of the present disclosure; a medium in which this *Corynebacterium glutamicum* strain has been cultured; or a combination of two or more of these.

The composition of the present disclosure may further comprise arbitrary suitable excipients to be commonly used in compositions for amino acid production. Such excipients may be, for example, a preservative, a wetting agent, a dispersing agent, a suspending agent, a buffering agent, a stabilizer, or an isotonic agent, but are not limited thereto.

In the composition of the present disclosure, the variant, polynucleotide, vector, strain, medium, L-glutamic acid, and the like are as described in the other aspects.

### [Mode for Invention]

Hereinafter, the present disclosure will be described in more detail by way of Examples. However, the following Examples are only preferred embodiments for illustrating the present disclosure, and thus are not intended to limit the scope of the present disclosure thereto. Meanwhile, technical matters not described in the present specification can be sufficiently understood and easily implemented by those skilled in the technical field of the present disclosure or similar technical fields.

### Example 1: Construction of plasmid

A plasmid (pDCM2, FIG. 1, SEQ ID NO: 19) for the insertion and replacement of genes in the *Corynebacterium* chromosome was designed and synthesized using the Gene-synthesis service of BIONICS Co., Ltd. The plasmid was designed to contain restriction enzyme sites to be easily used for cloning by referring to a commonly known paper on the sacB system (Gene, 145 (1994) 69-73). The pDCM2 plasmid thus synthesized has the following properties:
1) The pDCM2 plasmid has a replication origin that works only in *E. coli,* and self-replication is thus possible in *E. coli* but not in *Corynebacterium.*
2) The pDCM2 plasmid has a kanamycin resistance gene as a selection marker.
3) The pDCM2 plasmid has a Levan sucrose gene (sacB) as a secondary positive-selection marker.
4) No gene information derived from the pDCM2 plasmid remains in the finally constructed strain.

### Example 2: Construction of vector for expressing variant of glucosamine-6-phosphate deaminase in microorganism

In order to explore the effect of the variant (A137V; SEQ ID NO: 1), in which the 137^{nd} amino acid (alanine) of the amino acid sequence of a glucosamine-6-phosphate deaminase(SEQ ID NO: 3) is substituted with valine, on L-glutamic acid production, a vector for the construction of a strain expressing the same was prepared as follows.

PCR was performed using the genomic DNA (gDNA) of wild-type *Corynebacterium glutamicum* ATCC13869 as a template along with a primer pair of SEQ ID NOS: 5 and 6, and a primer pair of SEQ ID NOS: 7 and 8, respectively. Then, overlapping PCR was performed using a mixture of the two fragments obtained above as a template along with a primer pair of SEQ ID NOS: 5 and 8 to obtain a fragment. The PCR was performed as follows: denaturation at 94°C for 5 minutes; 30 cycles of 94°C for 30 seconds, 55°C for 30 seconds, and 72°C for 1 minute 30 seconds; and 72°C for 5 minutes. The pDCM2 vector was treated with *sma*I and the PCR product obtained above was fusion-cloned thereinto. The fusion cloning was performed using the In-Fusion^{®} HD cloning kit (Clontech). The obtained plasmid was named pDCM2-nagB(A137V).

### Example 3: Construction of L-glutamic acid-producing strain derived from wild-type Corynebacterium glutamicum and construction of strain for introducing variant of glucosamine-6-phosphate deaminase

### Example 3-1: Construction of Corynebacterium glutamicum strain having L-glutamic-acid-producing ability derived from wild-type Corynebacterium glutamicum

In order to construct a strain having the L-glutamic acid-producing ability derived from wild-type *Corynebacterium glutamicum* ATCC13869, a *Corynebacterium glutamicum* ATCC13869 ΔodhA strain, in which the *odhA* gene is deleted, was constructed based on the literature (Appl Environ Microbiol. 2007 Feb;73(4):1308-19. Epub 2006 Dec 8.).

Specifically, for the deletion of the *odhA* gene, PCR was performed using chromosomal DNA of *Corynebacterium glutamicum* ATCC13869 as a template along with a primer pair of SEQ ID NOS: 11 and 12 and a primer pair of SEQ ID NOS: 13 and 14, respectively. Then, overlapping PCR was performed using a mixture of the two fragments obtained above as a template along with a primer pair of SEQ ID NOS: 11 and 14 to obtain a fragment. The PCR was performed as follows: denaturation at 94°C for 5 minutes; 30 cycles of 94°C for 30 seconds, 55°C for 30 seconds, and 72°C for 1 minute and 30 seconds; and 72°C for 5 minutes. The pDCM2 vector was treated with *sma*I and the PCR product obtained above was fusion-cloned thereinto. The fusion cloning was performed using the In-Fusion^{®} HD cloning kit (Clontech). The thus-obtained plasmid was named pDCM2-ΔodhA.

After transforming the constructed pDCM2-ΔodhA vector into the *Corynebacterium glutamicum* ATCC13869 strain by electroporation, a secondary crossover process was performed to obtain a strain in which the *odhA* gene is deleted on the chromosome. The presence of gene deletion was confirmed through PCR and genome sequencing using SEQ ID NOS: 15 and SEQ ID NO: 16, and the constructed strain was named ATCC13869ΔodhA.

The primer sequences used herein are shown in Table 1 below.

**[Table 1]**

| SEQ ID NO | Name | Sequence |
|---|---|---|
| 11 | odhA_up_F | TGAATTCGAGCTCGGTACCCTTGAACGGAATTGGGTGG |
| 12 | odhA_up_R | CCCAGGTGGCATCGGTACCTTCACCCAGCGCCACGCAG |
| 13 | odhA_down_ F | CGCTGGGTGAAGGTACCGATGCCACCTGGGTTGGTCAAG |
| 14 | odhA_down_ R | GTCGACTCTAGAGGATCCCCGGACAAGGAATGGAGAGA |
| 15 | odhA_del_F | CTTACCGTTGTTGCCCTT |
| 16 | odhA_del_R | CTCCTTCACCCACATCATT |

### Example 3-2: Construction of strain for expressing variant of glucosamine-6-phosphate deaminase

The vector constructed in Example 2 was transformed into ATCC13869ΔodhA constructed in Example 3-1.

Strains into which a variant was introduced were selected using SEQ ID NOS: 9 and 10 among the strains in which the homologous recombination occurred. Each selected strain was named ATCC13869 ΔodhA_nagB_A137V.

### Example 3-3: Comparison of L-glutamic acid-producing ability of strains for expression of variant of glucosamine-6-phosphate deaminase

In order to examine the L-glutamic acid-producing ability of the strains constructed in Example 3-2, these strains were cultured using the following method with the ATCC13869ΔodhA strain as the control.

Each strain was inoculated into a 250 mL corner-baffle flask containing 25 mL of a seed medium, and incubated at 30°C for 20 hours with shaking at 200 rpm. Then, 1 mL of the seed culture solution was inoculated into a 250 mL corner-baffle flask containing 25 mL of a production medium and cultured with shaking at 30°C for 40 hours at 200 rpm. After completion of the culture, L-glutamic acid-producing ability was measured using high performance liquid chromatography (HPLC) and the measurement results are shown in Table 2 below. The concentration and concentration increase rate of glutamic acid in the culture medium for each strain experimented are shown in Table 2 below.

### <Seed Medium>

glucose 1%, beef extract 0.5%, polypeptone 1%, sodium chloride 0.25%, yeast extract 0.5%, urea 0.2%, pH 7.2

### <Production Medium>

raw sugar 6%, calcium carbonate 5%, ammonium sulfate 2.25%, potassium monophosphate 0.1%, magnesium sulfate 0.04%, iron sulfate 10 mg/L, thiamine hydrochloride 0.2 mg/L, biotin 50 µg/L

**[Table 2]**

| Strain Name | Concentration of L-Glutamic Acid (g/L) | Increase Rate of Concentration of L-Glutamic Acid (%) |
|---|---|---|
| ATCC13869 ΔodhA | 1.9 | - |
| ATCC13869 Δ | 2.6 | 36.8 |
| odhA_nagB_A137V | | |

As shown in Table 2 above, it was confirmed that the concentration of L-glutamic acid was significantly increased in the ATCC13869 ΔodhA_nagB_A137V strain, into which the *nagB*(A137V) gene was introduced, compared to the ATCC13869 ΔodhA strain.

The ATCC13869 ΔodhA_nagB_A137V strain was named CA02-1611, and it was deposited with the Korean Culture Center of Microorganisms (KCCM), a depositary under the Budapest Treaty, on November 30, 2020, and was given an Accession Number KCCM12852P.

From the above description, those skilled in the technical field to which the present disclosure belongs will understand that the present disclosure may be implemented in other specific forms without changing the technical spirit or essential features thereof. In this regard, it should be understood that the embodiments described above are illustrative in all respects and not limiting. The scope of the present disclosure should be construed as including all changes or modified forms derived from the meaning and scope of the claims to be described below rather than the above detailed description, and equivalent concepts thereof.

### [Accession Number]

Depository institution: Korea Culture Center of Microorganisms
Accession number: KCCM12852P
Accession date: 20201130

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA02-1611 | Accession number given by the
INTERNATIONAL DEPOSITARY AUTHORITY:
KCCM12852P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on November. 30. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: November. 30. 2020.

¹ Where Rule 6.4(d) applies, such date is the date on which the status of international depositary authority was
acquired.

## Claims

1. A glucosamine-6-phosphate deaminase variant consisting of an amino acid sequence represented by SEQ ID NO: 1, wherein alanine, which is an amino acid corresponding to position 137 of SEQ ID NO: 3, is substituted with valine.

2. A polynucleotide encoding the variant according to claim 1.

3. A *Corynebacterium glutamicum* strain comprising the variant according to claim 1 or a polynucleotide encoding the variant.

4. The strain according to claim 3, which has increased L-glutamic acid producing ability as compared to *Corynebacterium glutamicum* comprising a polypeptide of SEQ ID NO: 3 or a polynucleotide encoding the polypeptide.

5. A method for producing L-glutamic acid, the method comprising culturing a *Corynebacterium glutamicum* strain comprising the variant according to claim 1 or a polynucleotide encoding the variant in a medium.
